# EUROPÄISCHE PATENTSCHRIFT

(11) **EP 1 094 687 B1**
(45) Veröffentlichungstag und Bekanntmachung des Hinweises auf die Patenterteilung: **23.06.2010**
(21) Anmeldenummer: 00119019.8
(22) Anmeldetag: 01.09.2000
(51) Int. Cl.: H04R 25/00

(54) **Anordnung zum Ankoppeln eines Treibers an eine Ankoppelstelle der Ossikelkette**
Device for mechanical coupling of a driver to a coupling part of the ossicullar chain
Dispositif pour le couplage mécanique d'un vibreur à un place de couplage du chaîne ossicullair

(30) Priorität: 07.10.1999 DE 19948336
(43) Veröffentlichungstag der Anmeldung: 25.04.2001
(73) Patentinhaber: PHONAK AG, 8712 Stäfa (CH)
(72) Erfinder: Müller, Gerd M.,Dr.rer.nat (Dr. Dipl.-Phys.), 85716 Lohhof (DE)
(74) Vertreter: Schwan, Gerhard

(56) Entgegenhaltungen:
- EP-A- 0 901 779
- WO-A-99/07436

## Beschreibung

Die Erfindung betrifft eine implantierbare Anordnung zum mechanischen Ankoppeln eines zu mechanischen Schwingungen anregbaren ausgangsseitigen Treiberteils eines aktiven oder passiven Hörsystems an eine vorgewählte Ankoppelstelle an der Ossikelkette, der Steigbügelfußplatte oder einer das runde Fenster oder ein artifizielles Fenster in der Cochlea, im Vestibulum oder im Labyrinth (Gleichgewichtsorgan) abschließenden Membran über eine Koppelanordnung, die eine vom Treiberteil in mechanische Schwingungen versetzbare Koppelstange sowie ein mit der vorgewählten Ankoppelstelle in Verbindung bringbares Koppelelement aufweist, wobei die Koppelstange und das Koppelelement über wenigstens eine Kupplung miteinander verbunden sind und zumindest ein im implantierten Zustand an der Ankoppelstelle anliegender Abschnitt des Koppelelements zur verlustarmen, d.h. im Wesentlichen schallharten, Schwingungseinleitung in die Ankoppelstelle ausgelegt ist.

Es sind teil- oder vollimplantierbare aktive Hörsysteme für eine direkte mechanische Stimulation bekannt. Bei solchen Hörsystemen wird das Schallsignal mit einem Sensor (Mikrofon) in ein elektrisches Signal umgewandelt und in einer elektronischen Signalverarbeitungsstufe verstärkt; dieses verstärkte elektrische Signal wird einem implantierten elektromechanischen Wandler zugeführt, dessen ausgangsseitige mechanische Schwingungen unmittelbar, also mit direktem mechanischen Kontakt, dem Mittel- beziehungsweise Innenohr zugeführt werden. Dies gilt unabhängig davon, ob eine reine Innenohrschwerhörigkeit bei vollständig intaktem Mittelohr oder eine kombinierte Schwerhörigkeit (Mittel- und Innenohr geschädigt) rehabilitiert werden soll. Daher sind in der jüngeren wissenschaftlichen und Patent-Literatur implantierbare elektromechanische Wandler sowie Verfahren zur direkten Ankopplung der mechanischen Wandlerschwingungen an das intakte Mittelohr beziehungsweise das Innenohr zur Rehabilitation einer reinen Innenohrschwerhörigkeit sowie auch an verbleibende Ossikel des Mittelohres bei artifiziell oder pathologisch verändertem Mittelohr zur Versorgung einer Schalleitungsschwerhörigkeit sowie deren Kombinationen beschrieben worden.

Als elektromechanisches Wandlerverfahren kommen grundsätzlich alle physikalischen Wandlungsprinzipien in Frage wie elektromagnetisch, elektrodynamisch, magnetostriktiv, dielektrisch und piezoelektrisch. Verschiedene Forschungsgruppen haben sich in den letzten Jahren im wesentlichen auf zwei dieser Verfahren konzentriert: elektromagnetisch und piezoelektrisch. Eine Übersicht über diese Wandlervarianten findet sich bei Zenner und Leysieffer (HNO 1997 Vol. 45, 749 - 774).

Beim piezoelektrischen Verfahren ist eine mechanisch direkte Kopplung der ausgangsseitigen Wandlerschwingungen an die Mittelohrossikel oder direkt an das ovale Fenster notwendig. Beim elektromagnetischen Prinzip kann die Kraftkopplung einerseits über einen Luftspalt erfolgen ("kontaldos"), das heißt, nur ein Permanentmagnet wird durch dauerhafte Fixation in direkten mechanischen Kontakt mit einem Mittelohrossikel gebracht. Andererseits besteht die Möglichkeit, den Wandler vollständig in einem Gehäuse zu realisieren (wobei Spule und Magnet mit kleinstmöglichem Luftspalt gekoppelt sind) und die ausgangsseitigen Schwingungen über ein mechanisch steifes Koppelelement mit direktem Kontakt auf die Mittelohrossikel zu übertragen (Leysieffer et al. 1997 (HNO 1997, Vol. 45, pp. 792-800).

In der Patentliteratur finden sich einige der oben genannten Realisierungsvarianten sowohl elektromagnetischer wie auch piezoelektrischer Hörgerätewandler: US-A-5 707 338 (Adams et al.), WO 98/06235 (Adams et al.), WO 98/06238 (Adams et al.), WO 98/06236 (Kroll et al.), WO 98/06237 (Bushek et al.), US-A-5 554 096 (Ball), US-A-3 712 962 (Epley), US-A-3 870 832 (Fredrickson), US-A-5 277 694 (Leysieffer et al.), DE-C-198 40 211 (Leysieffer), DE-A-198 40 212.0 (Leysieffer), US-A-5 015 224 (Maniglia), US-A-3 882 285 (Nunley), US-A-4 850 962 (Schaefer).

Das teilimplantierbare, piezoelektrische Hörsystem der japanischen Gruppe um Suzuki und Yanigahara setzt für eine Implantation des Wandlers das Fehlen der Mittelohrossikel und eine freie Paukenhöhle voraus, um das Piezoelement an den Stapes ankoppeln zu können (Yanigahara et al.: Efficacy of the partially implantable middle ear implant in middle and inner ear disorders. Adv. Audiol.., Vol. 4, Karger Basel (1988), pp. 149-159. SUZUKI et al.: Implantation of partially implantable middle ear implant and the indication. Adv. Audiol., Vol. 4, Karger Basel (1988), pp. 160-166). Ebenso wird bei dem Verfahren eines implantierbaren Hörsystems für Innenohrschwerhörige nach US-A-4 850 962 (Schaefer) grundsätzlich der Amboss entfernt, um ein piezoelektrisches Wandlerelement an den Stapes ankoppeln zu können. Dies gilt im wesentlichen auch für weitere Entwicklungen, die auf der SCHAEFER-Technologie basieren und in den oben genannten Patentschriften dokumentiert sind (US-A-5 707 338, WO 98/06235, WO 98/06238, WO 98/06236, WO 98/06237).

Der elektromagnetische Wandler nach Ball ("*Floating Mass Transducer FMT*", US-A-5 624 376, US-A-5 554 096) wird dagegen bei intaktem Mittelohr mit Titanclips direkt an dem langen Fortsatz des Amboss fixiert. Der elektromagnetische Wandler des teilimplantierbaren Systems nach FREDRICKSON (FREDRICKSON et al.: Ongoing investigations into an implantable elektromagnetic hearing aid for moderate to severe sensorineural hearing loss. Otolaryngologic Clinics Of Nord America, Vol. 28/1 (1995), pp. 107-121) wird bei ebenfalls intakter Ossikelkette des Mittelohres mechanisch direkt an den Ambosskörper gekoppelt. Das Gleiche gilt für die piezoelektrischen und elektromagnetischen Wandler nach LEYSIEFFER (Leysieffer et al.: Ein implantierbarer piezoelektrischer Hörgerätewandler für Innenohrschwerhörige. HNO 1997/45, pp. 792-800, DE 41 04 358.8, DE 198 40 211.2, DE 198 40 212,0). Auch bei dem elektromagnetischen Wandlersystem nach MANIGLIA (Manigila et al.: Contactless semi-implantable electromagnetic middle ear device for the treatment of senrorineural hearing loss. Otolaryngologic Clinics Of Nord America, Vol. 28/1 (1995), pp. 121-141) wird bei intakter Ossikelkette ein Permanentmagnet mechanisch an der Ossikelkette dauerhaft fixiert, der jedoch über eine Luftspaltkopplung von einer Spule mechanisch angetrieben wird.

Bei den beschriebenen Wandler- und Ankopplungsvarianten sind grundsätzlich zwei Implantationsprinzipien zu unterscheiden:
a) Einerseits befindet sich der elektromechanische Wandler mit seinem aktiven Wandlerelement selbst im Mittelohrbereich in der Paukenhöhle und ist dort direkt mit einem Ossikel oder dem Innenohr verbunden (US-A-4 850 962, US-A-5 015 225, US-A-5 707 338, WO 98/06235, WO 98/06238, WO 98/06236, WO 98/06237, US-A-5 624 376, US-A-5 554 096).
b) Andererseits befindet sich der elektromechanische Wandler mit seinem aktiven Wandlerelement außerhalb des Mittelohrbereiches in einer artifiziell geschaffenen Mastoidhöhle; die ausgangsseitigen mechanischen Schwingungen werden dann mittels mechanisch passiver Koppelelemente über geeignete operative Zugänge (natürlicher aditus ad antrum, Eröffnung des chorda-facialis-Winkels oder über eine artifizielle Bohrung vom Mastoid aus) zum Mittel- beziehungsweise Innenohr übertragen (FREDRICKSON et al.: Ongoing investigations into an implantable elektromagnetic hearing aid for moderate to severe sensorineural hearing loss. Otolaryngologic Clinics Of Nord America, Vol. 28/1 (1995), pp. 107-121; DE 41 04 358.8, ältere Anmeldung DE 198 40 211.2, ältere Anmeldung DE 198 40 212.0).

Bei den Varianten nach a) kann der Wandler als sogenannter "Floating Mass"-Wandler ausgeführt sein, d.h., das Wandlerelement benötig keine "Reaktio" über eine feste Verschraubung mit dem Schädelknochen, sondern es schwingt aufgrund von Massenträgheitsgesetzen mit seinem Wandlergehäuse und überträgt diese direkt auf ein Mittelohrossikel (US-A-5 624 376, US-A-5 554 096, US-A-5 707 338, WO 98/06236). Dies bedeutet einerseits, dass vorteilhaft auf ein implantierbares Fixationssystem an der Schädelkalotte verzichtet werden kann; andererseits bedeutet diese Variante nachteilig, dass voluminöse artifizielle Elemente in die Paukenhöhle eingebracht werden müssen und deren Langzeit- und Biostabilität insbesondere bei temporären pathologischen Veränderungen des Mittelohres (z.B. otitis media) heute nicht bekannt beziehungsweise gewährleistet sind. Ein weiterer wesentlicher Nachteil besteht darin, dass die Wandler vom Mastoid aus mit ihrer elektrischen Zuleitung ins Mittelohr gebracht und dort mit Hilfe geeigneter operativer Werkzeuge fixiert werden müssen; dies erfordert einen erweiterten Zugang durch den chorda-facialis-Winkel und bringt somit eine latente Gefährdung des in unmittelbarer Nachbarschaft gelegenen Gesichtsnerven (nervus facialis) mit sich.

Bei den Wandlervarianten nach b) wird das Wandlergehäuse mit implantierbaren Positionier- und Fixationssystemen an der Schädelkalotte befestigt (vorteilhafte Ausführung DE-A-196 18 964 entsprechend US-A-5 788 711). Sowohl bei dem teilimplantierbaren System nach FREDRICKSON (Ongoing investigations into an implantable elektromagnetic hearing aid for moderate to severe sensorineural hearing loss. Otolaryngologic Clinics Of North America, Vol. 28/1 (1995), pp. 107-121) wie auch bei dem vollimplantierbaren Hörsystem nach LEYSIEFFER und ZENNER (HNO 1998, Vol. 46, 853-863 und 844-852) wird bei der Ankopplung des schwingenden Treiberteils an den Ambosskörper zur dauerhaften und mechanisch sicheren Schwingungsübertragung davon ausgegangen, dass die Spitze der Koppelstange, die in die laserinduzierte Vertiefung des Mittelohrossikels eingebracht wird, langfristig eine Osseointegration erfährt, das heißt, die Koppelstange verwächst fest mit dem Ossikel und gewährleistet so eine sichere Übertragung dynamischer Druck- und Zugkräfte. Dieser Langzeiteffekt ist zur Zeit jedoch noch nicht wissenschaftlich nachgewiesen beziehungsweise gesichert. Weiterhin besteht bei dieser Ankopplungsart bei einem technischen Wandlerdefekt der Nachteil, dass eine Entkopplung vom Ossikel zur Entfernung des Wandlers nur mit mechanisch basierten operativen Methoden vorgenommen werden kann, was eine erhebliche Gefährdung des Mittelohres und insbesondere des Innenohres bedeuten kann.

Der wesentliche Vorteil dieser Wandlerausführungsformen nach b) besteht jedoch darin, dass das Mittelohr weitgehend frei bleibt und der Koppelzugang zum Mittelohr ohne größeres Gefährdungspotential des nervus facialis erfolgen kann. Ein vorzugsweises operatives Verfahren hierzu ist in US-A-6 077 215 beschrieben. Grundlegende vorteilhafte Formen passiver Koppelelemente zur Übertragung der ausgangsseitigen Wandlerschwingungen vom Mastoid aus zum Mittel- beziehungsweise Innenohr sind in EP-A-0 499 940 sowie in EP-A-0 901 779 und in HNO 1998 Vol. 46, 27-37 - Lehner et al.: "Kaltfließende Elemente zur Ankopplung eines implantierbaren Hörgerätewandlers an Gehörknöchelchen oder Perilymphe" beschrieben. Dabei handelt es sich insbesondere um Koppelelemente aus Gold, vorzugsweise weichgeglühtem Feingold, in Form eines C-Bandes für den langen Ambossfortsatz, einer Bandschlaufe für den langen Ambossfortsatz und eines Glöckchens für das Steigbügelköpfchen, wobei sich diese Koppelelemente unter Verwendung von ohrchirurgischen Standardinstrumenten ankoppeln und erforderlichenfalls auch wieder lösen lassen.

Die ältere EP-Patentanmeldung 00 105 227.3 beschreibt eine implantierbare Anordnung zum mechanischen Ankoppeln eines zu mechanischen Schwingungen anregbaren ausgangsseitigen Treiberteils eines aktiven oder passiven Hörsystems an eine vorgewählte Ankoppelstelle an der Ossikelkette, der Steigbügelfußplatte oder einer das runde Fenster oder ein artifizielles Fenster in der Cochlea, im Vestibulum oder im Labyrinth (Gleichgewichtsorgan) abschließenden Membran über eine Koppelanordnung, die ein mit der vorgewählten Ankoppelstelle in Verbindung bringbares Koppelelement aufweist. Ein im implantierten Zustand an der Ankoppelstelle anliegendes Dämpfungsglied mit entropieelastischen Eigenschaften führt zu einer schallweichen Ankopplung und Verminderung des Risikos einer Beschädigung der natürlichen Strukturen im Bereich der Ankoppelstelle während und nach der Implantation.

Aus der WO 99/08475 ist ein aktives Hörsystem bekannt, bei dem ein kapazitiver Sensor Schwingungen des Malleus in ein elektrisches Signal umwandelt, welches nach Durchlaufen einer elektronischen Schaltung in einen Stimulator eingespeist wird, der seinerseits das Innenohr mechanisch oder elektrisch stimuliert. Der kapazitive Sensor umfasst eine erste Elektrode, die mit dem Malleus über eine Kugelgelenkkupplung schwenkbar gekoppelt ist sowie eine zweite am Mastoid entweder starr oder ebenfalls mittels einer Kugelgelenkkupplung schwenkbar festgelegte Elektrode. Die Kugelgelenkkupplung ist dabei so ausgelegt, dass die beiden Elektroden sich mit Bezug aufeinander frei ausrichten können, selbst wenn die Schwingungsrichtung des Malleus sich beispielsweise als Funktion der Frequenz ändert.

Eine Anordnung nach dem Oberbegriff des Anspruchs 1 ist in der DE 197 38 587 C1 beschrieben, bei der die erste Kupplungshälfte im Wesentlichen stabförmig und die zweite Kupplungshälfte etwa hülsenförmig ausgebildet ist, wobei durch Verschieben und/oder Verdrehen der beiden Kupplungshälften die Relativlage von Koppelstange und Koppelelement am Implantationsort in situ einstellbar ist. Die beiden Bauteile werden in der eingestellten Relativlage zuverlässig und langzeitstabil fixiert, indem mittels eines Crimpwerkzeugs eine Crimpkraft auf die hülsenförmige zweite Kupplungshälfte aufgebracht wird, wodurch sich diese plastisch kaltverformt, wohingegen die stabförmige erste Kupplungshälfte unter der wirkenden Crimpkraft keiner plastischen Kaltverformung unterzogen wird.

Neben den erläuterten aktiven Hörsystemen sind auch passive Hörsysteme in Form von Prothesen als Totalersatz (TORP = total ossicular replacement prosthesis) oder als Teilersatz (PORP = partial ossicular replacement prosthesis) für die Ossikelkette bekannt (D. I. Bojrab et al. "Ossiculoplasty with composite prostheses" in Otolaryngologic Clinics of North America, Vol. 27, No. 4, 1994, pp. 759-776). Bei solchen passiven Systemen bildet das Trommelfell selbst oder ein Bereich des noch intakten, dem Trommelfell zugewandten "Rests" der Ossikelkette das ausgangsseitige Treiberteil. So ist in der US 5 370 689 eine passive Mittelohrprothese als Stapesersatz offenbart, welche einen länglichen Stababschnitt umfasst, dessen eines Ende mit der Stapesfußplatte verbunden ist. Am anderen Ende des Stababschnitts ist ein offener Ring angeformt, der über das freie Ende des langen Ambossfortsatzes geschoben wird. Die Bewegung des vom langen Ambossfortsatz angetriebenen Stapesersatzes wird mittels einer Zugvorrichtung modifiziert, an welcher der Stapesmuskel angreift. Zu diesem Zweck ist die Zugvorrichtung auf den Stababschnitt aufgeschoben, wobei die Passung zwischen dem Stababschnitt und der Innenfläche der Zugvorrichtung so gewählt ist, dass beide Teile während der Implantation relativ zueinander bewegt werden können, wohingegen eine unerwünschte axiale oder rotatorische Bewegung der Zugvorrichtung nach der Implantation ausgeschlossen ist.

Der vorliegenden Erfindung liegt die Aufgabe zugrunde, eine Anordnung der eingangs genannten Art zu schaffen, die es erlaubt, die Relativlage der beiden Kupplungshälften der Kupplung am Implantationsort in situ möglichst feinfühlig in vielen Freiheitsgraden einzustellen, wobei die eingestellte Relativlage nach der Implantation auf einfache Weise zuverlässig und langzeitstabil aufrechterhalten bleibt.

Diese Aufgabe wird bei einer Anordnung nach dem Oberbegriff des Anspruchs 1 dadurch gelöst, dass eine erste Kupplungshälfte der Kupplung eine Außenkontur mit mindestens näherungsweise der Gestalt einer Kugelkalotte aufweist, die in einer zur Außenkontur wenigstens teilweise komplementären Innenkontur einer zweiten Kupplungshälfte aufnehmbar ist, wobei die Kupplung gegen Reibkräfte reversibel verschwenk- und/oder drehbar, jedoch bei im implantierten Zustand auftretenden dynamischen Kräften im Wesentlichen starr ist.

Die erfindungsgemäße Anordnung sorgt auf besonders einfache und gleichwohl zuverlässige Weise dafür, dass die beiden Kupplungshälften während der Implantation in eine gewünschte Relativlage gebracht werden können, wobei dennoch nach der Implantation die von dem Treiberteil ausgehenden, in die erste oder zweite Kupplungshälfte eingeleiteten mechanischen Schwingungen im Wesentlichen starr auf die jeweils andere Kupplungshälfte übertragen werden, ohne dass es hierfür irgendeines zusätzlichen Arbeitsschritts bedarf Ausgehend von den vorgegebenen, bekannten dynamischen Kräften, die im implantierten Zustand von der Kupplung übertragen werden müssen, und den höheren Kräften, die im Zuge der Implantation vom Operateur typischerweise aufgebracht werden, wählt man die die Eigenschaften der Paarung der beiden Kupplungshälften wesentlich beeinflussenden Parameter wie Werkstoff, Oberflächenrauheit (Mikrogeometrie) und Passung (Makrogeometrie) dergestalt, dass insbesondere der Stick-Slip-Effekt und die Reibungskräfte zwischen den Kupplungshälften bei den als Zweites genannten Kräften ein problemloses statisch reversibles Verstellen der Kupplung während der Implantation ermöglichen, wohingegen die Kupplung bei den zuerst genannten dynamischen Kräften starr ist.

Vorteilhafte Weiterbildungen der Erfindung sind Gegenstand der Unteransprüche.

Die Handhabung der Anordnung lässt sich wesentlich vereinfachen, wenn die Kupplung für ein reversibles An- und Abkuppeln ausgelegt ist.

Die zweite Kupplungshälfte der Kupplung kann zumindest zwei Federarme aufweisen, mittels derer die erste Kupplungshälfte wenigstens teilweise umgreifbar ist. Die Federarme, die stoffschlüssig, beispielsweise durch Löten, Schweißen oder dergleichen, verbunden oder aber auch einstückig ausgeführt sein können, sind vorzugsweise nach innen federnd vorgespannt an die erste Kupplungshälfte anlegbar.

Ferner kann die zweite Kupplungshälfte der Kupplung auch etwa glockenförmige Gestalt aufweisen und insbesondere mehrere im Wesentlichen senkrecht zur Umfangsrichtung verlaufende Schlitze umfassen, die sich bis zu einer der ersten Kupplungshälfte zugewandten Stirnseite der zweiten Kupplungshälfte erstrecken. Auf diese Weise lässt sich die erste Kupplungshälfte sicher in der zweiten Kupplungshälfte aufnehmen. Gleichzeitig wird eine ausreichende Flexibilität der zweiten Kupplungshälfte für ein reversibles An- und Abkoppeln geschaffen.

In vorteilhafter weiterer Ausgestaltung der Erfindung ist eine zweite Kupplung vorgesehen, die gegen Reibkräfte reversibel linear und/oder rotatorisch verstellbar, jedoch bei im implantierten Zustand auftretenden dynamischen Kräften im Wesentlichen starr ist, wobei eine erste Kupplungshälfte eine Außenkontur mit mindestens näherungsweise zylindrischer, vorzugsweise kreiszylindrischer, Gestalt hat, die in einer zur Außenkontur wenigstens teilweise komplementären Innenkontur einer zweiten Kupplungshälfte aufnehmbar ist. Die zweite Kupplung ist vorzugsweise für ein reversibles An- und Abkoppeln ausgelegt und lässt sich sowohl zwischen der Kupplung, bei der die erste Kupplungshälfte näherungsweise die Gestalt einer Kugelkalotte hat (erste Kupplung), und dem Treiberteil als auch zwischen der ersten Kupplung und der Ankoppelstelle positionieren. Es ist bevorzugt, dass im implantierten Zustand eine Übertragung von dynamischen Kräften zwischen den beiden Kupplungshälften der zweiten Kupplung im Wesentlichen in Richtung der Längsachse der ersten Kupplungshälfte erfolgt.

Ein besonders einfacher Aufbau ergibt sich, wenn die zweite Kupplungshälfte der zweiten Kupplung als Hülse ausgebildet ist. Die Hülse kann wenigstens einen im Wesentlichen in ihrer Längsrichtung verlaufenden Schlitz, der sich zumindest über einen Teil der Hülsenlänge erstreckt, aufweisen. Ferner kann sich zur Erhöhung der Flexibilität wenigstens ein Schlitz bis zu einer der ersten Kupplungshälfte zugewandten Stirnseite der Hülse erstrecken.

Erstreckt sich ein Schlitz über die gesamte Hülsenlänge, so kann die Wandung im Bereich der beiden Schlitzufer des durchgehenden Schlitzes nach außen weisen und einen Einführabschnitt bilden, in den die erste Kupplungshälfte im Wesentlichen senkrecht zu ihrer Längsachse einführbar ist.

Wenigstens ein Schlitz kann zumindest einseitig in einer die Elastizität der zweiten Kupplungshälfte sowie deren Sicherheit gegen Beschädigung erhöhenden Entlastungsöffnung enden, deren Begrenzungslinie die beiden Schlitzufer verbindet, wobei die Entlastungsöffnung quer zur Schlitzrichtung eine Abmessung aufweist, die größer als diejenige des Schlitzes ist.

Die Begrenzungslinie mindestens einer Entlastungsöffnung kann die Schlitzufer bogenförmig, vorzugsweise im Wesentlichen kreisbogenförmig, verbinden oder aber in Form eines im Wesentlichen senkrecht zum Schlitz verlaufenden Querschlitzes ausgebildet sein.

Bei einer weiteren Ausführungsform der Erfindung ist wenigstens ein Abschnitt einer Wandung der Hülse der zweiten Kupplung nach innen federnd vorgespannt an die erste Kupplungshälfte anlegbar. Weiterhin können wenigstens zwei Schlitze vorgesehen sein, wobei zumindest ein sich zwischen zwei benachbarten Schlitzen befindender Abschnitt einer Wandung der Hülse nach innen federnd vorgespannt an die erste Kupplungshälfte anlegbar ist. Dabei kann vorgesehen sein, wenigstens zwei benachbarte Schlitze endseitig insbesondere im Wesentlichen U-förmig miteinander zu verbinden, so dass eine federnde Zunge entsteht.

Zur Erleichterung des An- und Abkoppelvorgangs lässt sich die Außenkontur der ersten Kupplungshälfte der zweiten Kupplung im Bereich ihres der zweiten Kupplungshälfte zugewandten Endes mit einem sich in Richtung auf das Ende hin verjüngenden Einführbereich versehen.

Zu diesem Zweck kann auch die Innenkontur der zweiten Kupplungshälfte mindestens einer Kupplung im Bereich ihres der ersten Kupplungshälfte zugewandten Endes mit einem sich in Richtung auf das Ende hin erweiternden Einführbereich versehen sein. Dies gilt sowohl für die erste als auch für die zweite Kupplung.

Mindestens eine erste und/oder eine zweite Kupplungshälfte wenigstens einer Kupplung lassen sich überdies vorteilhaft mit dem zugeordneten Koppelelement oder der zugeordneten Koppelstange einstückig verbinden.

Die erfindungsgemäße Anordnung kann Teil eines aktiven, teil- oder vollimplantierbaren Hörsystems sein, bei dem das ausgangsseitige Treiberteil ein schwingfähiges Teil, insbesondere eine schwingfähige Membran, eines elektromechanischen Hörgeratewandlers ist. Die Anordnung nach der Erfindung kann aber auch Teil eines passiven Hörsystems, insbesondere einer Teil- oder Voll-Mittelohrprothese, sein, bei dem im implantierten Zustand das Trommelfell als ausgangsseitiges Treiberteil genutzt ist.

Bevorzugte Ausführungsbeispiele der Anordnung nach der Erfindung werden nachstehend anhand der beiliegenden Zeichnungen naher erläutert. Es zeigen:
- Fig. 1: in vergrößertem Maßstab schematisch einen implantierten Hörgerätewandler sowie eine Koppelanordnung mit einer vom Hörgerätewandler angetriebenen Koppelstange und einem Koppelelement, welches letzteres zum einen über eine Kupplung mit der Koppelstange verbunden und zum anderen an die Ossikelkette angekoppelt ist;
- Fig. 2: in noch größerem Maßstab eine schematische perspektivische Darstellung des Hörgerätewandlers nach Fig. 1, der über eine abgewandelte Koppelanordnung an den Ambosskörper angekoppelt ist;
- Fig. 3: eine schematische vergrößerte Ansicht des mit einem Kreis III versehenen Bereichs der Fig. 2;
- Fign. 4 und 5: schematische Darstellungen einer Ausführungsform, bei der die Kupplung zwischen der Koppelstange und dem Koppelelement als Kugelgelenkkupplung ausgebildet ist;
- Fig. 6: eine schematische perspektivische Darstellung eines abgewandelten Koppelelements mit geänderter Kugelgelenkkupplung;
- Fign. 7 bis 11: schematische perspektivische Darstellungen von abgewandelten Koppelanordnungen mit zwei Kupplungen, wobei eine erste Kupplung als Kugelgelenkkupplung und eine zweite Kupplung als Steckkupplung ausgebildet ist;
- Fign. 12 bis 17: schematische perspektivische Darstellungen weiterer abgewandelter Steckkupplungen; und
- Fig. 18: eine schematische perspektivische Darstellung einer erfindungsgemäßen passiven Mittelohrprothese mit Kugelgelenkkupplung.

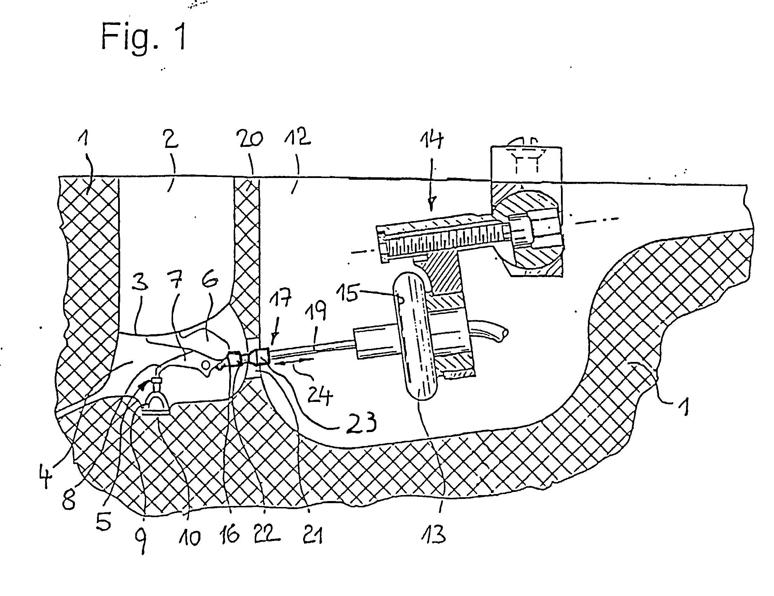

In Fig. 1 ist ein Teil eines menschlichen Schädelknochens 1 mit dem Gehörgang 2, dem davon durch das Trommelfell 3 abgetrennten Mittelohrraum (Paukenhöhle) 4 und der in der Paukenhöhle befindlichen Ossikelkette 5 dargestellt. Zu der Ossikelkette 5 gehören der Hammer 6, der Amboss 7 mit dem langen Ambossfortsatz 8 sowie der Steigbügel 9 mit der Steigbügelfußplatte 10. In einer artifiziellen Mastoidhöhle 12 ist ein elektromechanischer Hörgerätewandler 13 mittels eines insgesamt mit 14 bezeichneten Positionier- und Fixiersystems fixiert. Der Hörgerätewandler 13 kann beispielsweise als Piezowandler zur vibratorischen Stimulation der Ossikelkette insbesondere in der aus US-A-5 277 694 bekannten Weise aufgebaut sein, und er ist Bestandteil eines mindestens teilimplantierbaren und vorzugsweise vollimplantierbaren Hörgerätes, beispielsweise eines Hörgerätes der aus HNO 1997 Vol. 45, 749 - 774 bekannten Art.

Zum mechanischen Ankoppeln eines zu mechanischen Schwingungen anregbaren, in Fig. 1 nur schematisch angedeuteten ausgangsseitigen Treiberteils 15 des Hörgerätewandlers 13, insbesondere einer schwingfähigen Membran dieses Wandlers, an eine vorgewählte Ankoppelstelle 16 an der Ossikelkette 5, beispielsweise an den "glatten" Körper des Ambosses 7, von Mastoidseite aus, ist ein Schwingungsübertragungsweg in Form einer biokompatiblen, mechanisch passiven Koppelanordnung 17 vorgesehen. Die Koppelanordnung 17 ist mit dem aktiv schwingfähigen ausgangsseitigen Treiberteil 15 verbunden, und sie liegt im implantierten Zustand mit einem von dem Hörgerätewandler 13 abliegenden Ankoppelende an der Ankoppelstelle 16 an. Wird an den Hörgerätewandler 13 eine elektrische Spannung angelegt, wird die Koppelanordnung 17 mittels des ausgangsseitigen Treiberteils 15 zu vibratorischen Schwingungen in Axialrichtung der Koppelanordnung veranlasst. Infolgedessen führen die von einem (nicht dargestellten) eingangsseitigen Wandler (Mikrofon) aufgenommenen und elektrisch gewandelten Audiosignale nach elektronischer Verstärkung in einem Elektronikmodul des aktiven Hörsystems unmittelbar zu mechanischen Auslenkungen der Koppelanordnung 17. Diese Auslenkungen entsprechen der akustischen Information. Die Auslenkungen der Koppelanordnung 17 werden an die Ossikelkette 5 des Mittelohrs bzw. an den Steigbügel 9, die Steigbügelfußplatte 10 oder eine nicht dargestellte Membran weitergeleitet, die das ovale beziehungsweise runde Fenster oder ein artifizielles Fenster in der Cochlea, im Vestibulum oder im Labyrinth (Gleichgewichtsorgan) abschließt. Die Auslenkungen der Koppelanordnung bewirken so bei entsprechender Auslegung des vorverarbeitenden elektronischen Systems einen audiologischen Verstärkungseffekt.

Die Koppelanordnung 17 weist eine mit dem ausgangsseitigen Treiberteil 15 mechanisch fest verbundene Koppelstange 19 auf, die bei der gezeigten Ausführungsform im Wesentlichen auf ihrer gesamten Länge die Gestalt eines geraden Zylinders hat. Die Koppelstange 19 reicht im implantierten Zustand von der Mastoidhöhle 12 aus bevorzugt durch einen in der hinteren Gehörgangswand 20 befindlichen natürlichen, erforderlichenfalls artifiziell erweiterten Knochendurchbruch (Aditus ad antrum) 21 hindurch in die Paukenhöhle 4. Zu der Koppelanordnung 17 gehört ferner ein Koppelelement 22, das mit dem von dem Hörgerätewandler 13 abliegenden Ende der Koppelstange 19 über eine Kupplung 23 verbunden und über ein Ankoppelende an der Ankoppelstelle 16 angekoppelt ist.

Die schematisch dargestellte Kupplung 23 umfasst zwei Kupplungshälften, von denen eine erste Kupplungshälfte eine Außenkontur mit mindestens näherungsweise der Gestalt einer Kugelkalotte aufweist, die in einer zur Außenkontur wenigstens teilweise komplementären Innenkontur einer zweiten Kupplungshälfte aufnehmbar ist. Die erste Kupplungshälfte ist am freien Ende der Koppelstange 19 vorzugsweise einstückig angeformt. Dabei ist die Kupplung 23 so ausgebildet, dass sie vom Operateur bei der Implantation gegen Reibkräfte reversibel verschwenk- und drehbar jedoch bei im implantierten Zustand auftretenden dynamischen Kräften im Wesentlichen starr ist. Somit ist eine feinfühlige Anpassung der Relativlage von Koppelstange 19 und Koppelelement 22 an die Gegebenheiten des Implantationsorts in situ möglich, wobei sich die einmal eingestellte Relativlage nach der Implantation durch die dann auftretenden dynamischen Kräfte nicht mehr verstellt.

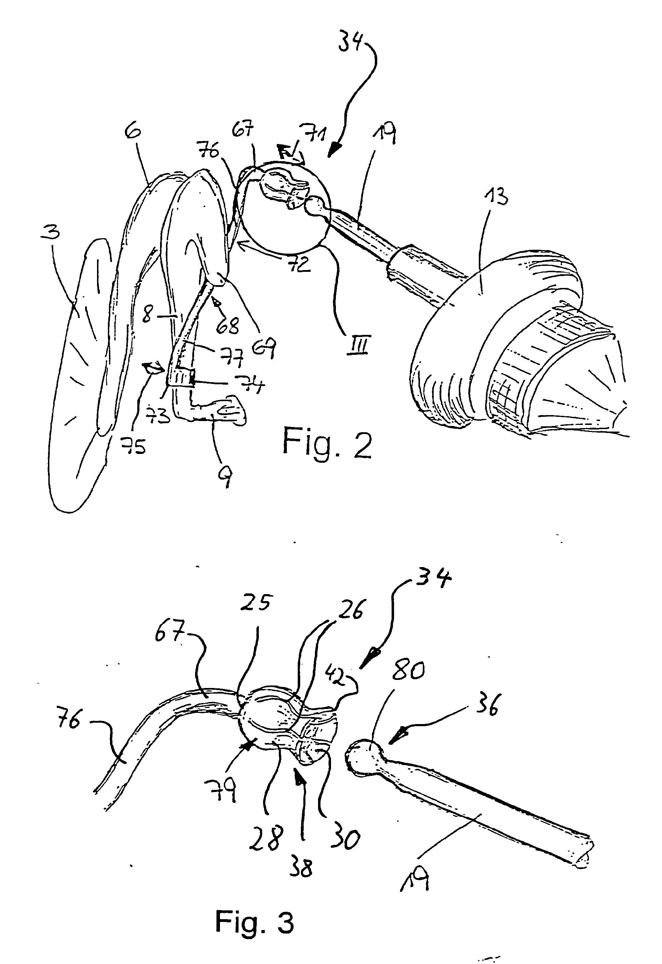

In den Fign. 2 und 3 ist eine Koppelanordnung mit einer Kupplung 34 gezeigt, die als Kugelgelenkkupplung ausgebildet ist und deren erste Kupplungshälfte 36 einen Kugelkopf 80 aufweist, der in eine zweite Kupplungshälfte 38 in Form einer Kugelaufhahme 79 eingeführt werden kann. Die Kugelaufnahme 79 besitzt etwa glockenförmige Gestalt mit mehreren Schlitzen 26, die sich von einer der ersten Kupplungshälfte 36 zugewandten Stirnseite 42 aus im Wesentlichen senkrecht zur Umfangsrichtung der Kugelaufnahme 79 erstrecken. Auf diese Weise werden Federarme 28 gebildet, die beim An- und Abkuppelvorgang reversibel nach außen federn können, wobei ein sich in Richtung auf die Stirnseite 42 nach außen erweiternder Einführbereich 30 das Ankuppeln des Kugelkopfs 80 an der Kugelaufnahme 79 erleichtert.

Während der Kugelkopf 80 einstückig mit der Koppelstange 19 ausgebildet ist, geht die Kugelaufnahme 79 über ein Anschlussstück 25 in ein koppelstangenseitiges Ende 67 eines Koppelelements 68 über. Das Koppelelement 68 ist als zweiarmiger Hebel mit den beiden Armen 76 und 77 ausgebildet, der sich in einem mittleren Bereich an dem kurzen Ambossfortsatz 69 abstützt, Wird das koppelstangenseitige Ende 67 des Arms 76 mittels der Koppelstange 19 zu einer Bewegung entsprechend dem Doppelpfeil 71 veranlasst, schwenkt das Koppelelement 68 um einen von dem kurzen Ambossfortsatz 69 bestimmten Drehpunkt 72. Dadurch wird ein am Arm 77 angeordnetes Ankoppelende 73 des Koppelelements 68, das mit dem langen Ambossfortsatz 8 über eine Federklammer 74 oder dergleichen in Eingriff steht, gemäß dem Doppelpfeil 75 verstellt. Durch entsprechende Bemessung der relativen Längen der Arme 76 und 77 des Koppelelements 68 kann ein gewünschtes Hebelverhältnis eingestellt werden. Die Kupplung 34 ist in situ nicht nur dreh- und schwenkbar, sie kann am Implantationsort auch an- und abgekuppelt werden, wodurch sich die Handhabbarkeit der Vorrichtung wesentlich erleichtert. Nach der Implantation wird die eingestellte Relativlage von Koppelstange 19 und Koppelelement 68 durch die auftretenden dynamischen Kräfte nicht mehr verstellt.

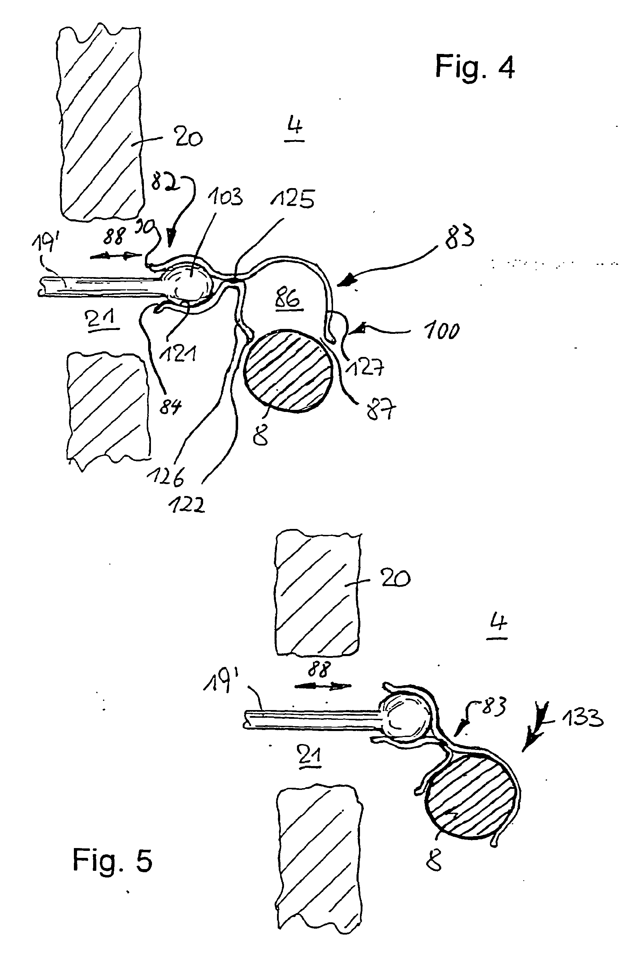

Bei einer abgewandelten Ausführungsform gemäß den Fign. 4 und 5 ist eine Kupplung 82 in Form einer Kugelgelenkkupplung vorgesehen, deren erste Kupplungshälfte eine Kugel 103 umfasst, die an einem dem Hörgerätewandler 13 abgewandten Ende einer Koppelstange 19' einstückig mit der letzteren verbunden ist. Als Koppelelement 83 kommt eine federnde Klammer aus zwei bei 125 miteinander verbundenen, vorzugsweise verschweißten, Federarmen 126 und 127 zum Einsatz. Die Federarme 126, 127 bilden einerseits eine zweite Kupplungshälfte in Form einer Kugelaufnahme 121 für die Kugel 103 der Koppelstange 19' sowie andererseits eine Aufnahmeöffnung 86 mit einem aufspreizbaren Durchlass 87 für das Zielossikel 8. Zur Erleichterung des Ankoppelvorgangs zwischen den beiden Kupplungshälften der Kupplung 82 ist die Kugelaufhahme 121 mit einem Einführbereich 84 versehen, der sich in Richtung auf eine Stirnseite 90 hin erweitert, wobei der Durchlass 87 für das Zielossikel 8 in einem Ankoppelende 100 des Koppelelements 83 plaziert ist, dessen Stirnseite 122 im Wesentlichen senkrecht zur Stirnseite 90 gelegen ist.

Das Koppelelement 83 kann mittels der Koppelstange 19' durch den Durchbruch 21 in der hinteren Gehörgangswand 20 hindurch in den Mittelohrraum 4 eingeführt und so positioniert werden, dass der aufspreizbare Durchlass 87 mit dem Zielossikel, beispielsweise dem langen Ambossfortsatz 8, entsprechend Fig. 4 ausgerichtet ist. Dann wird das Koppelelement 83 niedergedrückt und dadurch in Richtung des Pfeils 133 in Fig. 5 mit Bezug auf die Koppelstange 19' geschwenkt, bis das Zielossikel 8 unter Aufweitung des Durchlasses 87 in der Aufhahmeöffnung 86 liegt. Auf diese Weise wird eine sichere Ankopplung an das Zielossikel erreicht. Im implantierten Zustand führt die Koppelstange 19' Schwingungen im Wesentlichen in Richtung des Doppelpfeils 88 aus, wobei die Kupplung 82 die Schwingungen starr übertragt.

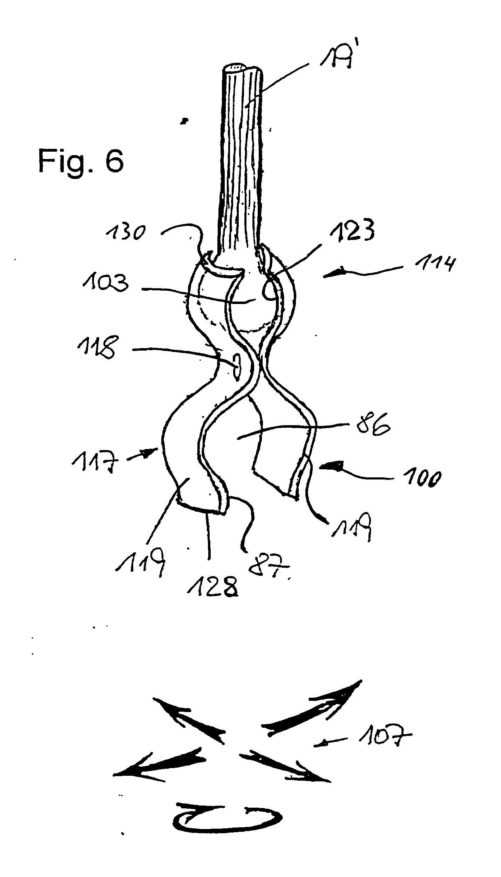

Ein in Fig. 6 dargestelltes Koppelelement 117 umfasst zwei bei 118 verschweißte gewellte Federarme 119, die auf der einen Seite der Verbindungsstelle 118 eine Kugelaufnahme 123 für die Kugel 103 der Koppelstange 19' sowie auf der anderen Seite dieser Verbindungsstelle den aufspreizbaren Durchlass 87 und die Aufnahmeöffnung 86 für das Zielossikel bilden. Das Koppelelement 117 lässt sich gegenüber der Koppelstange 19' gemäß der Pfeilgruppe 107 drehen und schwenken und unterscheidet sich vom Koppelelement 83 nach den Fign. 4 und 5 im Wesentlichen dadurch, dass die Kugelaufnahme 123 als zweite Kupplungshälfte einer Kupplung 114 keinen dem Einführbereich 84 entsprechenden Abschnitt aufweist und demgemäß nicht reversibel an- und abkuppelbar ist. Ferner liegt eine den Durchlass 87 für das Zielossikel aufnehmende Stirnseite 128 im Gegensatz zur Ausführungsform der Fign. 4 und 5 etwa parallel zu einer die zweite Kupplungshälfte mit der Kugelaufnahme 123 begrenzenden Stirnseite 130. Auch die Kupplung 114 überträgt die im implantierten Zustand auftretenden dynamischen Kräfte starr.

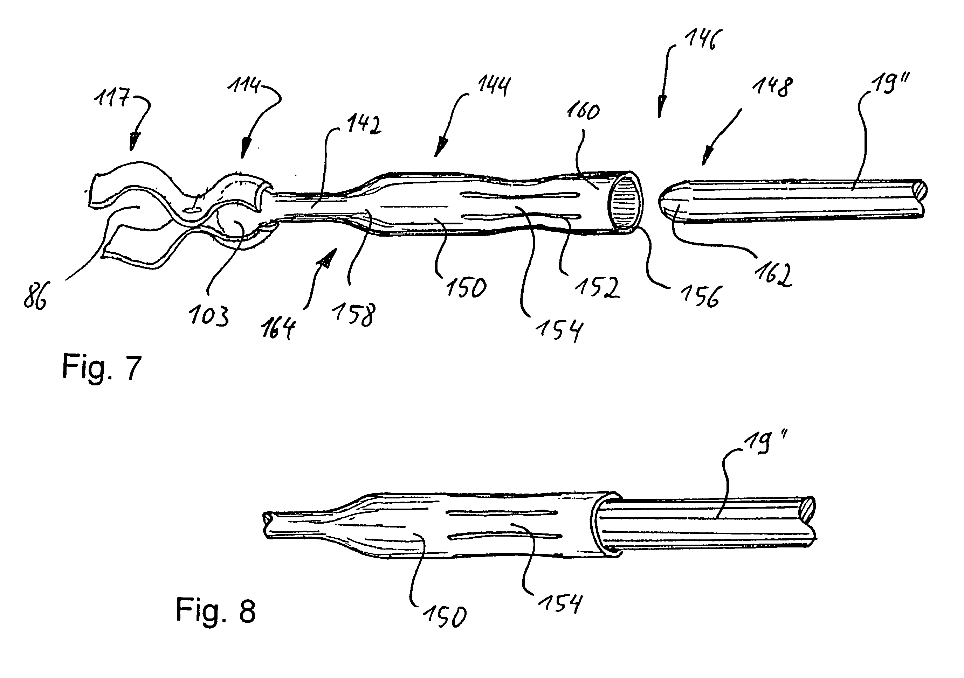

Bei einer abgewandelten Ausführungsform der erfindungsgemäßen Anordnung nach den Fign. 7 und 8 sind eine erste und eine zweite Kupplung in Reihe geschaltet, wobei als erste Kupplung die Kupplung 114 der Fig. 6 eingesetzt ist. Anstelle mit der Koppelstange 19' ist die Kugel 103 jedoch mit einem Stiel 142 eines insgesamt mit 164 bezeichneten Zwischenelements verbunden, welcher Stiel 142 über einen sich erweiternden Abschnitt 158 an einer Hülse 150 einer zweiten Kupplungshälfte 144 der zweiten Kupplung 146 festgelegt ist. Eine erste Kupplungshälfte 148 der Kupplung 146 wird von einem freien Ende einer Koppelstange 19'' gebildet, deren zweites Ende mit dem Hörgerätewandler 13 verbunden ist und von diesem in Schwingungen versetzt wird. Um das dem Hörgerätewandler 13 abgewandte freie Ende der Koppelstange 19'' leichter in die Hülse 150 der als Steckkupplung ausgelegten zweiten Kupplung 146 einführen zu können, ist am freien Ende der Koppelstange 19'' ein sich auf das Ende hin verjüngender Einführbereich 162 vorgesehen. Die Hülse 150 weist in ihrer Wandung 160 mehrere, im Wesentlichen in Längsrichtung der Hülse 150 verlaufende Schlitze 152 auf, die etwa gleichmäßig um den Umfang verteilt sind und vor einer Stirnseite 156 am freien Ende der Hülse 150 enden. Zwischen je zwei benachbarten Schlitzen 152 ist ein Steg 154 ausgebildet, der bei in die Hülse 150 eingesteckter erster Kupplungshälfte 148 an dieser nach innen vorgespannt mit vorgegebener Anpresskraft anliegt.

Die Reihenschaltung zweier Kupplungen hat insbesondere den Vorteil, dass bei der Implantation die Anordnung durch Lösen der Kupplung 146 zunächst in zwei getrennt zu handhabende Baugruppen unterteilt werden kann, die nach Ankopplung des Koppelelements 117 mittels seiner Aufnahmeöffnung 86 am Zielossikel wieder verbunden werden. Die Kupplung 146 lässt sich vom Operateur reversibel an- und abkuppeln und dabei in situ in Längsrichtung der Hülse 150 verschieben und um die Längsachse der Hülse 150 drehen, wobei die Auslegung der Kupplung 146 so getroffen ist, dass die vom Operateur eingestellte rotatorische sowie translatorische Relativlage der beiden Kupplungshälften 144 und 148 bei den im implantierten Zustand auftretenden dynamischen Kräften stabil aufrechterhalten bleibt, zumindest solange eine vorgegebene Mindesteinführtiefe der ersten Kupplungshälfte 148 in die zweite Kupplungshälfte 144 nicht unterschritten wird. Auch die nachfolgend beschriebenen weiteren Kugelgelenk- und Steckkupplungen sind bei der Implantation gegen Reibkräfte statisch reversibel verstellbar, übertragen jedoch die im implantierten Zustand auftretenden, niedrigeren dynamischen Kräfte starr.

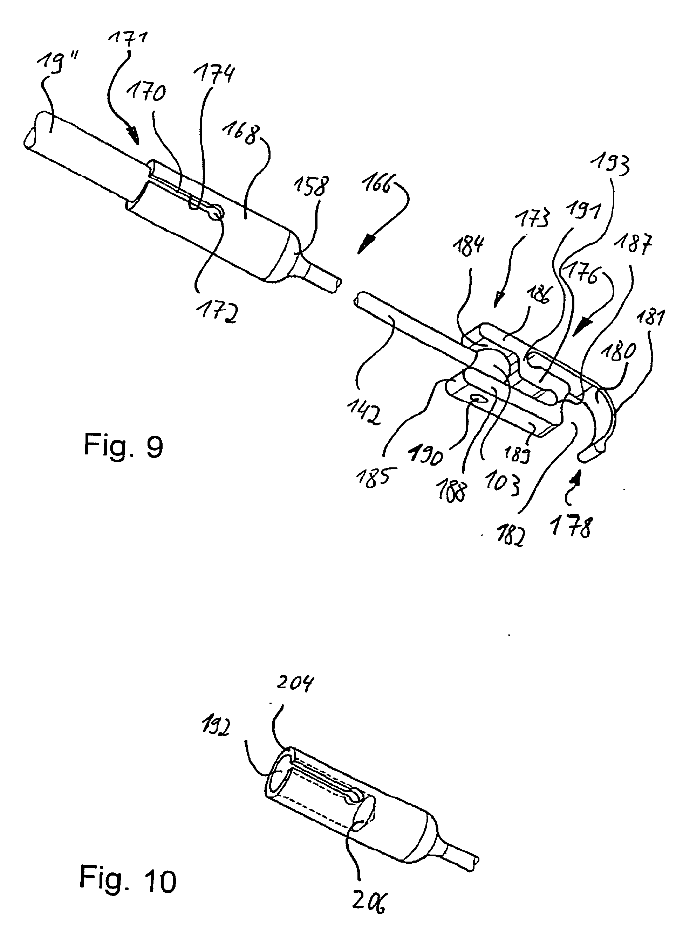

In den Fign. 9 und 10 ist ein weiteres Ausführungsbeispiel einer Anordnung veranschaulicht, bei welcher ebenfalls zwei in Reihe geschaltete Kupplungen eingesetzt sind, und zwar eine als Steckkupplung ausgebildete Kupplung 171 und eine Kupplung 173 in Form einer Kugelgelenkkupplung. Ein Zwischenelement 166 unterscheidet sich vom Zwischenelement 164 der Fign. 7 und 8 im Wesentlichen nur dadurch, dass die zweite Kupplungshälfte der Kupplung 171 eine abgewandelte Hülse 168 umfasst, die mit einem einzelnen Schlitz 170 versehen ist, der sich ausgehend von einer Stirnseite 204 am freien Ende der Hülse 168 in Hülsenlängsrichtung erstreckt und in einer im Wesentlichen runden Entlastungsöffnung 172 endet, welche die beiden Schlitzufer 174 verbindet. Die Steckkupplung 171 ist so ausgelegt, dass die an der Koppelstange 19'' vorgesehene erste Kupplungshälfte 148 in eine Aufnahme 192 der Hülse 168 stets soweit eingeschoben wird, dass das freie Ende der ersten Kupplungshälfte 148 an einem Tiefenanschlag 206 im Inneren der Hülse 168 anstößt. Eine optische Sichtkontrolle des Steckvorgangs ist durch den Schlitz 170 möglich.

Ein Koppelelement 176 ist einteilig ausgebildet und umfasst als zweite Kupplungshälfte der Kupplung 173 eine Kugelaufnahme 184, die von zwei sich gegenüberliegenden Federarmen 186 und 188 gebildet ist, welche sich beide bis zu einer kupplungsseitigen Stirnseite 185 des Koppelelements 176 erstrecken. Zur Erhöhung der Flexibilität des Federarms 188 ist dieser U-förmig in Richtung auf eine Aufnahmeöffnung 182 für das Zielossikel verlängert, wobei ein dem Federarm189 gegenüberliegender Schenkel 193 über einen Quersteg 191 mit dem Federarm 186 verbunden ist und ein zwischen dem Schenkel 193 und dem Federarm 189 vorgesehener Quersteg 187 so ausgebildet ist, dass seine von der Kugelaufnahme 184 wegweisende Außenfläche gemeinsam mit einer Innenfläche eines Federbügels 180 die Aufnahmeöffnung 182 bildet. Dabei ist der Federbügel 180 an der dem Federarm 186 gegenüberliegenden Seite des Querstegs 191 an demselben angeformt und verläuft zunächst in dünnwandiger Verlängerung des Federbügels 186 etwa parallel zu dem Schenkel 193, um dann in ein bogenförmiges Segment 181 überzugehen. Das freie Ende des Segments 181 endet im Wesentlichen auf gleicher Höhe wie eine Seitenfläche 189 des Federarms 188, so dass das Zielossikel etwa senkrecht mit Bezug auf die Seitenfläche 189 in die am Ankoppelende 178 des Koppelelements 176 vorhandene Aufnahmeöffnung 182 eingeführt wird. Die Seitenflache 189 ist etwa senkrecht mit Bezug auf eine die Stirnseite 185 aufnehmende Ebene ausgerichtet. In den Federarmen 186 und 188 ist jeweils ein Durchlass 190 dergestalt eingebracht, dass beide Durchlässe eine gemeinsame Längsachse aufweisen, welche überdies durch den Mittelpunkt der Kugel 103 verläuft. Auf diese Weise bilden die der Kugel 103 zugewandten Stirnseiten der Durchlässe 190 für die Kugel 103 eine definierte Anlagefläche, die in der Art einer Kugelpfanne ausgeformt sein kann. Das gesamte Koppelement 176 ist vorzugsweise aus Titan oder einer Titanlegierung gefertigt.

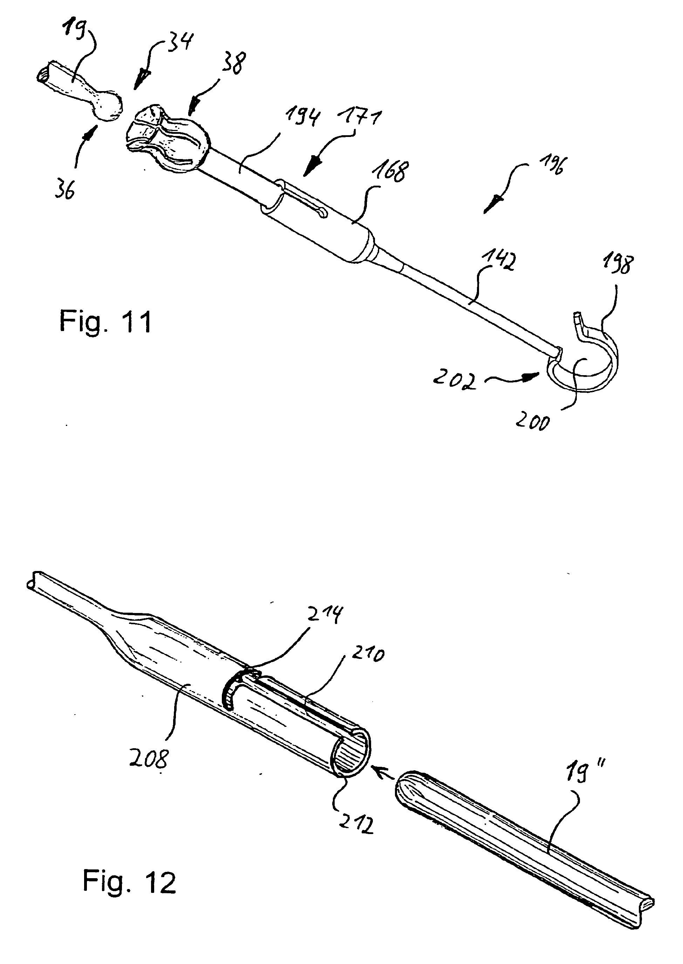

Fig. 11 gibt eine Anordnung wieder, bei der als Kugelgelenkkupplung die Kupplung 34 gemäß den Fign. 2 und 3 und als Steckkupplung die Kupplung 171 der Fign. 9 und 10 verwendet wird, wobei die zweite Kupplungshälfte 38 der Kupplung 34 mit einem Stiel 194 verbunden ist, dessen freies Ende die erste Kupplungshälfte der Kupplung 171 bildet. Im Gegensatz zur Ausführung nach den Fign. 9 und 10 endet der mit der Hülse 168 verbundene Stiel 142 jedoch nicht in der Kugel 103 sondern geht in ein Ankoppelende 202 für das Zielossikel über. Das Ankoppelende 202 umfasst eine Bandschlaufe 198, die eine Aufnahmeöffnung 200 für das Zielossikel bildet und beispielsweise um den langen Ambossfortsatz 8 herumlegbar ist. Für die Hülse 168 und den einstückig angeformten Stiel 142 wird als Werkstoff vorzugsweise Titan oder eine Titanlegierung eingesetzt, wohingegen die Bandschlaufe 198 insbesondere aus Gold oder einer Goldlegierung gefertigt wird.

Eine in Fig. 12 dargestellte Steckkupplung unterscheidet sich von der Kupplung 171 gemäß den Fign. 9 und 10 hauptsächlich dadurch, dass ein in einer Hülse 208 eingebrachter Schlitz 210 an seinem einer Stirnseite 212 abgewandten Ende nicht in einer runden Entlastungsöffnung entsprechend der Entlastungsöffnung 172 der Hülse 168, sondern in einem im Wesentlichen senkrecht zu dem Schlitz 210 angebrachten Querschlitz 214 endet.

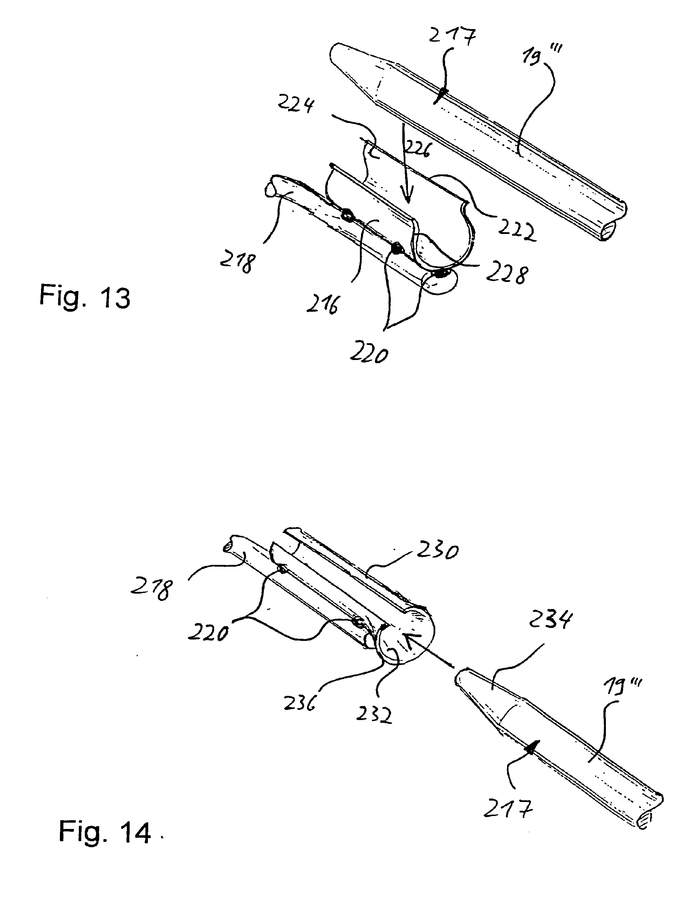

Weitere abgewandelte Steckkupplungen sind in den Fign. 13 bis 17 wiedergegeben. So wird bei einer Steckkupplung nach Fig. 13 eine an einem freien Ende einer Koppelstange 19''' angeformte erste Kupplungshälfte 217 in Richtung des Pfeils 226 im Wesentlichen senkrecht zu einer Längsachse einer als Hülse 216 ausgebildeten zweiten Kupplungshälfte eingeführt. Zu diesem Zweck ist die Hülse 216 mit einem in Längsrichtung durchgehenden Schlitz versehen, wobei eine Wandung 228 der Hülse 216 im Bereich von Schlitzufern 222 zu beiden Seiten des Schlitzes nach außen gewölbt ist, so dass sich ein Einführbereich 224 ergibt, der beim Ankoppeln der ersten Kupplungshälfte 217 mit einer Außenwand derselben wechselwirkt und ein Auffedern der Hülse 216 erleichtert. Die Hülse 216 ist an ihrer dem Schlitz etwa diametral gegenüberliegenden Außenseite mit einem Stiel 218 der Koppelanordnung über Verbindungsstellen 220 verbunden, insbesondere verschweißt oder verlötet.

Auch eine in Fig. 14 gezeigte Steckkupplung umfasst als zweite Kupplungshälfte eine Hülse 230 mit einem durchgehenden Schlitz, wobei jedoch im Unterschied zur Hülse 216 gemäß Fig. 13 ein sich nach außen erweiternder Einführbereich 232 an einer Stirnseite 236 eines freien Endes der Hülse 230 vorgesehen ist und die Wandung der Hülse 230 im Übrigen etwa kreiszylindrische Gestalt hat. Der Ankoppelvorgang der ersten Kupplungshälfte 217 an die Hülse 230 erfolgt im Wesentlichen in Längsrichtung der Hülse, wobei der Einführbereich 232 der Hülse 230 mit einem am freien Ende der ersten Kupplungshälfte 217 ausgebildeten, sich konisch verjüngenden Einführbereich 234 zusammenwirkt und ein Auffedern der Hülse 230 erleichtert.

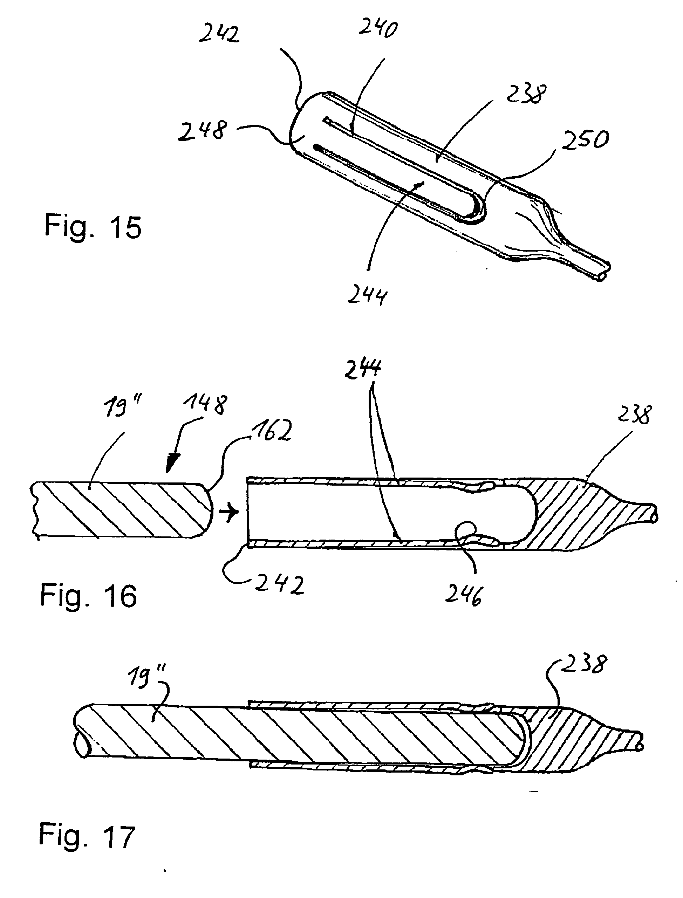

Die Fign. 15 bis 17 zeigen eine weitere abgewandelte Steckkupplung, die sich von der Kupplung 146 gemäß den Fign. 7 und 8 im Wesentlichen nur durch die Ausgestaltung von Schlitzen 240 in einer Wandung 248 der als Hülse 238 ausgebildeten zweiten Kupplungshälfte unterscheidet, Wie die Schlitze 152 in der Hülse 150 nach den Fign. 7 und 8 verlaufen auch die Schlitze 240 in der Hülse 238 im Wesentlichen in Hülsenlängsrichtung und enden vor einer Stirnseite 242 der Hülse 238. Insgesamt sind jedoch vier Schlitze 240 dergestalt in der Wandung 248 der Hülse 238 eingebracht, dass je zwei Schlitze 242 enger benachbart und an ihrem der Stirnseite 242 abgewandten Ende über ein im Wesentlichen U-förmiges Schlitzsegment 250 miteinander verbunden sind. Dabei werden zwei sich diametral gegenüberliegende federnde Zungen 244 gebildet, die nach innen federnd vorgespannt sind, wobei ein freies Ende der Zungen 244 bogenförmig nach innen übersteht und über eine Anlagefläche 246 an eine Außenfläche der ersten Kupplungshälfte 148 anlegbar ist.

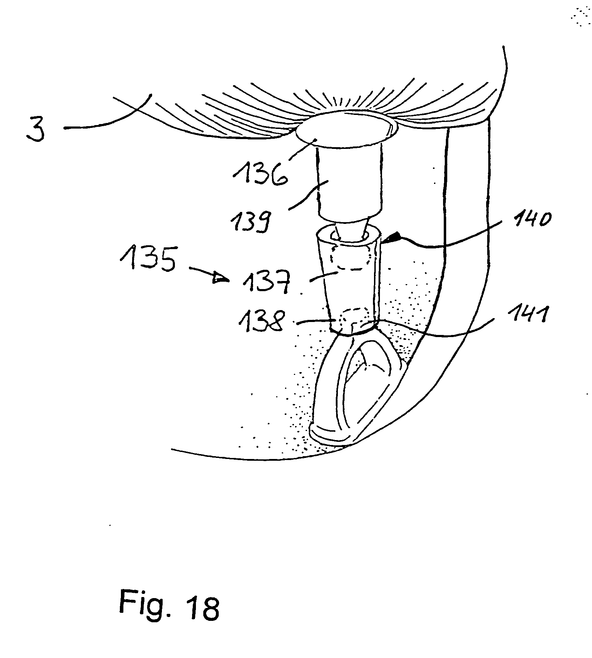

In Fig. 18 ist schematisch ein implantiertes passives Hörsystem dargestellt, bei dem als zu mechanischen Schwingungen anregbares ausgangsseitiges Treiberteil das Trommelfell 3 genutzt ist. Am Trommelfell 3 liegt eine insgesamt mit 135 bezeichnete TORP-Prothese (total ossicular replacement prosthesis) mit einem Kopf 136 an, der eine abgerundete Oberfläche aufweist. An den Kopf 136 schließt sich eine Koppelstange 139 an, die mit dem Kopf 136 einstückig verbunden sein kann und deren freies Ende über eine als Kugelgelenkkupplung ausgebildete Kupplung 140 mit einem freien Ende eines Koppelelements 137 verbunden ist. Ein von der Kupplung 140 abliegendes Ankoppelende 138 des Koppelelements 137 ist mit dem Steigbügelkopf 141 gekoppelt. Die Kupplung 140 gestattet bei der Implantation ein reversibles statisches Verschwenken und Verdrehen von Koppelelement 137 und Koppelstange 139 mit Bezug aufeinander, überträgt jedoch die im implantierten Zustand vom Trommelfell 3 in die Kupplung eingeleiteten dynamischen Kräfte starr. Der Kopf 136, die Koppelstange 139 und das Koppelelement 137 bestehen zweckmäßig aus einem implantierbaren metallischen oder keramischen Werkstoff.

Generell können als Werkstoffe für die Koppelstange, das Koppelelement, die Kupplung sowie das bedarfsweise zwischen zwei Kupplungen eingesetzte Zwischenelement alle bekannten biokompatiblen Metalle und ihre Legierungen verwendet werden, vor allem implantierfähiges Titan, insbesondere Reintitan mit einer Reinheit > 99,6 %. Daneben sind unter anderem Platin, Niob oder Tantal oder Legierungen von Titan, Platin, Niob beziehungsweise Tantal geeignet. Gegebenenfalls können die Koppelstange oder andere der oben genannten Bauteile aber auch aus einem implantierbaren keramischen Werkstoff, insbesondere Aluminiumoxid, bestehen. Ferner können aber auch langzeit-implantierbare Kunststoffe vorgesehen sein, so unter anderem vernetzte Silicone, Polyurethane, PTFE, FEP, Polycarbonate und dergleichen, die gegebenenfalls faserverstärkt, insbesondere kohlefaserverstärkt, sein können, wobei jedoch zumindest ein im implantierten Zustand an der Ankoppelstelle an der Ossikelkette, der Steigbügelfüßplatte oder einer das runde Fenster oder ein artifizielles Fenster in der Cochlea, im Vestibulum oder im Labyrinth (Gleichgewichtsorgan) abschließenden Membran anliegender Abschnitt des Koppelelements zur verlustarmen, d.h. im Wesentlichen schallharten, Schwingungseinleitung in die Ankoppelstelle ausgelegt ist und somit nur geringe Entropieelastizität aufweist. Dieser an der Ankoppelstelle anliegende Abschnitt des Koppelelements besteht vorzugsweise aus einem der genannten metallischen oder keramischen Werkstoffe oder ist in Gold oder einer Goldlegierung ausgeführt.

## Patentansprüche

1. Implantierbare Anordnung zum mechanischen Ankoppeln eines zu mechanischen Schwingungen anregbaren ausgangsseitigen Treiberteils (3, 15) eines aktiven oder passiven Hörsystems an eine vorgewählte Ankoppelstelle (16) an der Ossikelkette (5), der Steigbügelfußplatte (10) oder einer das runde Fenster oder ein artifizielles Fenster in der Cochlea, im Vestibulum oder im Labyrinth (Gleichgewichtsorgan) abschließenden Membran über eine Koppelanordnung (17), die eine vom Treiberteil (3, 15) in mechanische Schwingungen versetzbare Koppelstange (19, 19', 19", 19"') sowie ein mit der vorgewählten Ankoppelstelle (16) in Verbindung bringbares Koppelelement (22, 68, 83, 117, 137, 176, 196) aufweist, wobei die Koppelstange (19, 19', 19", 19"') und das Koppelelement (22, 68, 83, 117, 137, 176, 196) über wenigstens eine Kupplung (23, 34, 82, 114, 140, 146, 171, 173) miteinander verbunden sind und zumindest ein im implantierten Zustand an der Ankoppelstelle (16) anliegender Abschnitt (74, 119, 126, 127, 138, 180, 198) des Koppelelements (22, 68, 83, 117, 137, 176, 196) zur verlustarmen Schwingungseinleitung in die Ankoppelstelle (16) ausgelegt ist, **dadurch gekennzeichnet, dass** eine erste Kupplungshälfte (36, 103) der Kupplung (23, 34, 82, 114, 140, 173) eine Außenkontur mit mindestens näherungsweise der Gestalt einer Kugelkalotte aufweist, die in einer zur Außenkontur wenigstens teilweise komplementären Innenkontur einer zweiten Kupplungshälfte (38, 121, 123, 184) aufnehmbar ist, wobei die Kupplung (23, 34, 82, 114, 140, 173) gegen Reibkräfte reversibel verschwenk- und/oder drehbar, jedoch bei im implantierten Zustand auftretenden dynamischen Kräften im Wesentlichen starr ist.

2. Anordnung nach Anspruch 1, **dadurch gekennzeichnet, dass** die Kupplung (23, 34, 82, 173) für ein reversibles An- und Abkuppeln ausgelegt ist.

3. Anordnung nach Anspruch 1 oder 2, **dadurch gekennzeichnet, dass** die zweite Kupplungshälfte (38, 121, 123, 184) der Kupplung (34, 82, 114, 173) zumindest zwei Federarme (28, 119, 126, 127, 186, 188) aufweist, mittels derer die erste Kupplungshälfte (36, 103) wenigstens teilweise umgreifbar ist.

4. Anordnung nach Anspruch 3, **dadurch gekennzeichnet, dass** die Federarme (28, 119, 126, 127, 186, 188) nach innen federnd vorgespannt an die erste Kupplungshälfte (36, 103) anlegbar sind.

5. Anordnung nach einem der vorhergehenden Ansprüche, **dadurch gekennzeichnet, dass** die zweite Kupplungshälfte (38) der Kupplung (34) etwa glockenförmige Gestalt aufweist.

6. Anordnung nach Anspruch 5, **dadurch gekennzeichnet, dass** die zweite Kupplungshälfte (38) mehrere im Wesentlichen senkrecht zur Umfangsrichtung verlaufende Schlitze (26) aufweist, die sich bis zu einer der ersten Kupplungshälfte (36) zugewandten Stirnseite (42) der zweiten Kupplungshälfte (38) erstrecken.

7. Anordnung nach einem der vorhergehenden Ansprüche, **gekennzeichnet durch** eine zweite Kupplung (146, 171), die gegen Reibkräfte reversibel linear und/oder rotatorisch verstellbar, jedoch bei im implantierten Zustand auftretenden dynamischen Kräften im Wesentlichen starr ist, wobei eine erste Kupplungshälfte (148, 217) eine Außenkontur mit mindestens näherungsweise zylindrischer, vorzugsweise kreiszylindrischer, Gestalt hat, die in einer zur Außenkontur wenigstens teilweise komplementären Innenkontur einer zweiten Kupplungshälfte (144, 150, 168, 208, 216, 230, 238) aufnehmbar ist.

8. Anordnung nach Anspruch 7, **dadurch gekennzeichnet, dass** die zweite Kupplungshälfte der zweiten Kupplung (146, 171) als Hülse (150, 168, 208, 216, 230, 238) ausgebildet ist.

9. Anordnung nach Anspruch 8, **dadurch gekennzeichnet, dass** die Hülse (150, 168, 208, 216, 230, 238) wenigstens einen im Wesentlichen in ihrer Längsrichtung verlaufenden Schlitz (152, 170, 210, 240), der sich zumindest über einen Teil der Hülsenlänge erstreckt, aufweist.

10. Anordnung nach Anspruch 9, **dadurch gekennzeichnet, dass** sich wenigstens ein Schlitz (170) bis zu einer der ersten Kupplungshälfte (148) zugewandten Stirnseite 204) der Hülse (168) erstreckt.

11. Anordnung nach Anspruch 9 oder 10, **dadurch gekennzeichnet, dass** sich ein Schlitz über die gesamte Hülsenlänge erstreckt.

12. Anordnung nach einem der Ansprüche 9 bis 11, **dadurch gekennzeichnet, dass** wenigstens ein Schlitz (152, 240) vor einer Stirnseite (156, 242) der Hülse (150, 238) endet.

13. Anordnung nach einem der Ansprüche 9 bis 12, **dadurch gekennzeichnet, dass** wenigstens ein Schlitz (170, 210) zumindest einseitig in einer Entlastungsöffnung (172, 214) endet, deren Begrenzungslinie die beiden Schlitzufer (174) verbindet, wobei die Entlastungsöffnung (172, 214) quer zur Schlitzrichtung eine Abmessung aufweist, die größer als diejenige des Schlitzes (170, 210) ist.

14. Anordnung nach Anspruch 13, **dadurch gekennzeichnet, dass** die Begrenzungslinie mindestens einer Entlastungsöffnung (172) die Schlitzufer (174) bogenförmig, vorzugsweise im Wesentlichen kreisbogenförmig, verbindet.

15. Anordnung nach Anspruch 13 oder 14, **dadurch gekennzeichnet, dass** mindestens eine Entlastungsöffnung als im Wesentlichen senkrecht zum Schlitz (210) verlaufender Querschlitz (214) ausgebildet ist.

16. Anordnung nach einem der Ansprüche 8 bis 15, **dadurch gekennzeichnet, dass** wenigstens ein Abschnitt (154, 244) einer Wandung (160, 248) der Hülse (150, 168, 208, 216, 230, 238) nach innen federnd vorgespannt an die erste Kupplungshälfte (148, 217) anlegbar ist.

17. Anordnung nach einem der Ansprüche 8 bis 16, **dadurch gekennzeichnet, dass** wenigstens zwei Schlitze (152, 240) vorgesehen sind, wobei zumindest ein sich zwischen zwei benachbarten Schlitzen (152, 240) befindender Abschnitt (154, 244) einer Wandung (160, 248) der Hülse (150, 238) nach innen federnd vorgespannt an die erste Kupplungshälfte (148) anlegbar ist.

18. Anordnung nach Anspruch 17, **dadurch gekennzeichnet, dass** wenigstens zwei benachbarte Schlitze (240) endseitig insbesondere im Wesentlichen U-förmig miteinander verbunden sind.

19. Anordnung nach einem der Ansprüche 7 bis 18, **dadurch gekennzeichnet, dass** die Außenkontur der ersten Kupplungshälfte (148, 217) der zweiten Kupplung (146, 171) im Bereich ihres der zweiten Kupplungshälfte (144, 150, 168, 208, 216, 230, 238) zugewandten Endes mit einem sich in Richtung auf das Ende hin verjüngenden Einführbereich (162, 234) versehen ist.

20. Anordnung nach einem der Ansprüche 7 bis 19, **dadurch gekennzeichnet, dass** im implantierten Zustand eine Übertragung von dynamischen Kräften zwischen den beiden Kupplungshälften (148, 217; 144, 150, 168, 208, 216, 230, 238) der zweiten Kupplung (146, 171) im Wesentlichen in Richtung der Längsachse der ersten Kupplungshälfte (148, 217) erfolgt.

21. Anordnung nach einem der vorhergehenden Ansprüche, **dadurch gekennzeichnet, dass** die Innenkontur der zweiten Kupplungshälfte (38, 121, 184, 230) mindestens einer Kupplung (34, 82, 173) im Bereich ihres der ersten Kupplungshälfte (36, 103, 217) zugewandten Endes mit einem sich in Richtung auf das Ende hin erweiternden Einführbereich (30, 84, 232) versehen ist.

22. Anordnung nach einem der vorhergehenden Ansprüche, **dadurch gekennzeichnet, dass** mindestens eine erste (36, 103, 148, 217) und/oder eine zweite (184) Kupplungshälfte wenigstens einer Kupplung (34, 82, 114, 146, 173) mit dem zugeordneten Koppelelement (176) oder der zugeordneten Koppelstange (19, 19', 19", 19"') einstückig verbunden ist.

23. Anordnung nach einem der vorhergehenden Ansprüche, **dadurch gekennzeichnet, dass** sie Teil eines passiven Hörsystems ist, bei dem im implantierten Zustand das Trommelfell (3) als ausgangsseitiges Treiberteil genutzt ist.

## Claims

1. Implantable arrangement for mechanically coupling, via a coupling arrangement (17), an output-side driver part (3, 15) of an active or passive hearing system, the driver part being adapted to be excited to vibrate mechanically, to a preselected coupling site (16) at the ossicular chain (5), the foot plate (10) of the stapes or a membrane which closes the round window or an artificial window in the cochlear, the vestibulum or the labyrinth (equilibrium organ), wherein the coupling arrangement comprises a coupling rod (19, 19', 19", 19"'), which can be vibrated mechanically by the driver part (3, 15) and a coupling element (22, 68, 83, 117, 137, 176, 196) which can be connected to the preselected coupling site (16), wherein the coupling rod (19, 19', 19", 19"') and the coupling element (22, 68, 83, 117, 137, 176, 196) are connected via at least one coupling (23, 34, 82, 114, 140, 146, 171, 173) to each other and wherein at least one portion (74, 119, 126, 127, 138, 180, 198) of the coupling element (22, 68, 83, 117, 137, 176, 196), which portion adjoins the coupling site (16) in the implanted state, is designed for low-loss vibration excitation at the coupling site (16), characterize in that a first coupling half (36, 103) of the coupling (23, 34, 82, 114, 140, 173) has an at least approximately spherical outside contour, which is adapted to be received in an inside contour of a second coupling half (38, 121, 123, 184), the inside contour being at least partly complementary to said outside contour, wherein the coupling (23, 34, 82, 114, 140, 173) is adapted to swivel and/or rotate against friction forces in a reversible manner, while being substantially rigid with respect to dynamic forces occurring in the implanted state.

2. Arrangement according to claim 1, wherein the coupling (23, 34, 82, 173) is adapted to reversibly couple and decouple.

3. Arrangement according to claim 1 or 2, wherein the second coupling half (38, 121, 123, 184) of the coupling (34, 82, 114, 173) comprises at least two spring arms (28, 119, 136, 127, 186, 188) which are adapted to at least partially encompass the first coupling half (36, 103).

4. Arrangement according to claim 3, wherein the spring arms (28, 119, 126, 127, 186, 188) are elastically pretensioned into an inner direction to adjoin the first coupling half (36, 103).

5. Arrangement according to one of the preceding claims, wherein the second coupling half (38) of the coupling (34) is approximately bell-shaped.

6. Arrangement according to claim 5, wherein the second coupling half (38) comprises a plurality of slots (26) extending essentially perpendicular to the peripheral direction, which slots extend to a front face (42) of the second coupling half (38), which faces the first coupling half (36).

7. Arrangement according to one of the preceding claims, comprising a second coupling (146, 171), which is adapted to be linearly and/or rotationally adjusted in a reversible manner, while being substantially rigid with regard to dynamic forces occurring in the implanted state, wherein a first coupling half (148, 217) has an outside contour having an at least approximately cylindrical, preferably circular cylindrical, shape, which outside contour is adapted to be received in an inside contour of a second coupling half (144, 150, 168, 208, 216, 230, 238), which inside contour is at least partly complementary to said outside contour.

8. Arrangement according to claim 7, wherein the second coupling half of the second coupling (146, 171) is a sleeve (150, 168, 208, 216, 230, 238).

9. Arrangement according to claim 8, wherein the sleeve (150, 168, 208, 216, 230, 238) comprises at least one slot (152, 170, 210, 240) which extends substantially in the longitudinal direction of the sleeve and which runs along at least a portion of the length of the sleeve.

10. Arrangement according to claim 9, wherein at least one slot (170) extends to a front face (204) of the sleeve (168), which face faces the first coupling half (148).

11. Arrangement according to claim 9 or 10, wherein a slot extends along the entire length of the sleeve.

12. Arrangement according to one of claims 9 to 11, wherein at least one slot (152, 240) terminates in front of a front face (156, 242) of the sleeve (150, 238).

13. Arrangement according to one of claims 9 to 12, wherein at least one slot (170, 210) ends at least on one side in a relief opening (172, 214) having a boundary which connects the two sides (174) of the slot, and wherein the relief opening (172, 214) has a transverse dimension which is greater than that of the slot (170, 210).

14. Arrangement according to claim 13, wherein the boundary of at least one relief opening (172) connects the two sides (174) of the slot in an arc-shaped, preferably essentially circular, manner.

15. Arrangement according to claim 13 or 14, wherein at least one relief opening is designed as a transverse slot (214) extending substantially perpendicular to the slot (210).

16. Arrangement according to one of claims 8 to 15, wherein at least one portion (154, 254) of a wall (160, 248) of the sleeve (150, 168, 208, 216, 230, 238) is elastically pretensioned into an inner direction to adjoin the first coupling half (148, 217).

17. Arrangement according to one of claims 8 to 16, wherein at least two slots (152, 240) are provided, wherein at least a portion (154, 244) of a wall (160, 248) of the sleeve (150, 238) is elastically pretensioned into an inner direction to adjoin the first coupling half (148), with said portion being located between two adjacent slots (152, 240).

18. Arrangement according to claim 17, wherein the ends of at least two adjacent slots (240) are connected to each other, in particular in a substantially U-shaped manner.

19. Arrangement according to one of claims 7 to 18, wherein the outside contour of the first coupling half (148, 217) of the second coupling (146, 171) is provided, in the region of its end facing the second coupling half (144, 150, 168, 208, 216, 230, 238), with an insertion area (162, 234) which is tapered in the direction towards the end.

20. Arrangement according to one of claims 7 to 19, wherein, in the implanted state, transmission of dynamic forces between the two coupling halves (148, 217; 144, 150, 168, 208, 216, 230, 238) of the second coupling (146, 171) occurs substantially in the direction of the longitudinal axis of the first coupling half (148, 217).

21. Arrangement according to one of the preceding claims, wherein the inner contour of the second coupling half (38, 121, 184, 230) of at least one coupling (34, 82, 173) is provided, in the region of its end facing the first coupling half (36, 103, 217), with an insertion area (30, 84, 232) which is tapered in the direction towards the end.

22. Arrangement according to one of the preceding claims, wherein at least a first (36, 103, 148, 217) and/or a second (184) coupling half of at least one coupling (34, 82, 114, 146, 173) is connected with the associated coupling element (176) or the associated coupling rod (19, 19', 19", 19"') as a single piece.

23. Arrangement according to one of the preceding claims, wherein the arrangement is part of a passive hearing system, and wherein, in the implanted state, the ear drum (3) is used as an output side driver part.

## Revendications

1. Dispositif implantable pour le couplage mécanique d'une partie d'excitation (3, 15) côté sortie, pouvant être stimulée pour obtenir des vibrations mécaniques, d'un appareil auditif actif ou passif à une partie de couplage (16) présélectionnée sur la chaîne ossiculaire (5), la plaque de base de l'étrier (10) ou une membrane terminant la fenêtre ronde ou une fenêtre artificielle dans la cochlée, dans le vestibule ou dans le labyrinthe (organe d'équilibre) par l'intermédiaire d'un dispositif de couplage (17), qui présente une tige de couplage (19, 19', 19", 19"') pouvant être soumise par la partie d'excitation (3, 15) à des vibrations mécaniques et un élément de couplage (22, 68, 83, 117, 137, 176, 196) pouvant être mis en liaison avec le point de couplage (16) présélectionné, la tige de couplage (19, 19', 19", 19"') et l'élément de couplage (22, 68, 83, 117, 137, 176, 196) étant reliés l'un à l'autre par au moins un couplage (23, 34, 82, 114, 140, 146, 171, 173) et au moins une partie (74, 119, 126, 127, 138, 180, 198), s'appliquant dans l'état implanté sur le point de couplage (16), de l'élément de couplage (22, 68, 83, 117, 137, 176, 196) étant conçue pour l'application de vibrations pauvre en perte dans le point de couplage (16), **caractérisé en ce qu'**une première moitié d'accouplement (36, 103) de l'accouplement (23, 34, 82, 114, 140, 173) présente un contour extérieur avec au moins approximativement la forme d'une calotte sphérique, laquelle peut être réceptionnée dans un contour interne, au moins partiellement complémentaire par rapport au contour extérieur, d'une seconde moitié d'accouplement (38, 121, 123, 184), l'accouplement (23, 34, 82, 114, 140, 173) pouvant être basculé et/ou tourné de façon réversible par rapport à des forces de frottement, mais étant sensiblement rigide dans le cas de forces dynamiques apparaissant dans l'état implanté.

2. Dispositif selon la revendication 1, **caractérisé en ce que** l'accouplement (23, 34, 82, 173; est conçu pour un couplage et un découplage réversibles.

3. Dispositif selon la revendication 1 ou 2, **caractérisé en ce que** la seconde moitié d'accouplement (38, 121, 123, 184) de l'accouplement (34, 82, 114, 173) présente au moins deux bras de ressort (28, 119, 126, 127, 186, 188), au moyen desquels la première moitié d'accouplement (36, 103) peut être au moins en partie entourée.

4. Dispositif selon la revendication 3, **caractérisé en ce que** les bras de ressort (28, 119, 126, 127, 186, 188) peuvent être appliqués sur la première moitié d'accouplement (36, 103) en étant pré-tendus de façon élastique vers l'intérieur.

5. Dispositif selon l'une quelconque des revendications précédentes, **caractérisé en ce que** la seconde moitié d'accouplement (38) de l'accouplement (34) présente à peu près la forme d'une cloche.

6. Dispositif selon la revendication 5, **caractérisé en ce que** la seconde moitié d'accouplement (38) présente plusieurs fentes (26) agencées sensiblement perpendiculairement au sens périphérique, lesquelles fentes s'étendent jusqu'à un côté avant (42) tourné vers la première moitié d'accouplement (36), de la seconde moitié d'accouplement (38).

7. Dispositif selon l'une quelconque des revendications précédentes, **caractérisé par** un second accouplement (146, 171), qui peut être réglé de façon linéaire et/ou par rotation et réversible contre des forces de frottement, mais est sensiblement rigide dans le cas de forces dynamiques apparaissant dans l'état implanté, une première moitié d'accouplement (148, 217) ayant un contour extérieur avec une forme au moins approximativement cylindrique, de préférence cylindrique circulaire, qui peut être réceptionnée dans un contour intérieur, au moins en partie complémentaire par rapport au contour extérieur, d'une seconde moitié d'accouplement (144, 150, 168, 208, 216, 230, 238).

8. Dispositif selon la revendication 7, **caractérisé en ce que** la seconde moitié d'accouplement du second accouplement (146, 171) est conçue sous forme de manchon (150, 168, 208, 216, 230, 238).

9. Dispositif selon la revendication 8, **caractérisé en ce que** le manchon (150, 168, 208, 216, 230, 238) présente au moins une fente (152, 170, 210, 240) agencée sensiblement dans sa direction longitudinal, qui s'étend au moins sur une partie de la longueur de manchon.

10. Dispositif selon la revendication 9, **caractérisé en ce qu'**au moins une fente (170) s'étend jusqu'à un côté avant (204) tourné vers la première moitié d'accouplement (148), du manchon (168).

11. Dispositif selon la revendication 9 ou 10, **caractérisé en ce qu'**une fente s'étend sur l'ensemble de la longueur du manchon.

12. Dispositif selon l'une quelconque des revendications 9 à 11, **caractérisé en ce qu'**au moins une fente (152, 240) se termine avant un côté avant (156, 242) du manchon (150, 238).

13. Dispositif selon l'une quelconque des revendications 9 à 12, **caractérisé en ce qu'**au moins une fente (170, 210) se termine au moins d'un côté dans une ouverture de décharge (172, 214), dont la ligne de délimitation relie les deux bords de la fente (174), l'ouverture de décharge (172, 214) présentant transversalement au sens de la fente une dimension qui est supérieure à celle de la fente (170, 210).

14. Dispositif selon la revendication 13, **caractérisé en ce que** la ligne de délimitation d'au moins une ouverture de décharge (172) relie les bords de fente (174) en forme de coude, de préférence sensiblement en forme d'arc de cercle.

15. Dispositif selon la revendication 13 ou 14, **caractérisé en ce qu'**au moins une ouverture de décharge est conçue sous forme de fente transversale (214) agencée sensiblement perpendiculairement à la fente (210).

16. Dispositif selon l'une quelconque des revendications 8 à 15, **caractérisé en ce qu'**au moins une partie (154, 244) d'une paroi (160, 248) du manchon (150, 168, 208, 216, 230, 238) peut être appliquée contre la première moitié d'accouplement (148, 217), en étant pré-tendue vers l'intérieur de façon élastique.

17. Dispositif selon l'une quelconque des revendications 8 à 16, **caractérisé en ce qu'**au moins deux fentes (152, 240) sont prévues, au moins une partie (154, 244) se trouvant entre deux fentes (152, 240) voisines, d'une paroi (160, 248) du manchon (150, 238) pouvant être appliquée sur la première moitié d'accouplement (148), en étant pré-tendue vers l'intérieur de façon élastique.

18. Dispositif selon la revendication 17, **caractérisé en ce qu'**au moins deux fentes (240) voisines sont reliées l'une à l'autre côté extrémité en particulier sensiblement en forme de U.

19. Dispositif selon l'une quelconque des revendications 7 à 18, **caractérisé en ce que** le contour extérieur de la première moitié d'accouplement (148, 217) du second accouplement (146, 171) est doté dans la zone de son extrémité tournée vers la seconde moitié d'accouplement (144, 150, 168, 208, 216, 230, 238) d'une zone d'introduction (162, 234) se rétrécissant en direction de l'extrémité.

20. Dispositif selon l'une quelconque des revendications 7 à 19, **caractérisé en ce que**, dans l'état implanté, une transmission de forces dynamiques entre les deux moitiés d'accouplement (148, 217 ; 144, 150, 168, 208, 216, 230, 238) du second accouplement (146, 171) s'effectue sensiblement en direction de l'axe longitudinal de la première moitié d'accouplement (148, 217).

21. Dispositif selon l'une quelconque des revendications précédentes, **caractérisé en ce que** le contour intérieur de la seconde moitié d'accouplement (38, 121, 184, 230) d'au moins un accouplement (34, 82, 173) est doté dans la zone de son extrémité tournée vers la première moitié d'accouplement (36, 103, 217) d'une zone d'introduction (30, 84, 232) s'élargissant en direction de l'extrémité.

22. Dispositif selon l'une quelconque des revendications précédentes, **caractérisé en ce qu'**au moins une première moitié d'accouplement (36, 103, 148, 217) et/ou une seconde moitié de couplage (184) d'au moins un accouplement (34, 82, 114, 146, 173) est reliée d'une seule pièce à l'élément de couplage (176) attribué ou à la tige de couplage (19, 19', 19", 19"') attribuée.

23. Dispositif selon l'une quelconque des revendications précédentes, **caractérisé en ce qu'**il fait partie d'un appareil auditif passif, sur lequel, dans l'état implanté, la membrane du tympan (3) est utilisée comme partie d'excitation côté sortie.
